(19) Europäisches Patentamt
European Patent Office
Office européen des brevets

(11) Veröffentlichungsnummer: **0 301 245 B1**

(12) **EUROPÄISCHE PATENTSCHRIFT**

(45) Veröffentlichungstag der Patentschrift: **08.09.93**

(51) Int. Cl.[5]: **C07D 471/08**, A61K 31/435, //(C07D471/08,221:00,221:00)

(21) Anmeldenummer: **88110180.2**

(22) Anmeldetag: **25.06.88**

# (54) 3-Sulfonyl-3,7-diazabicyclo-(3,3,1)nonan-Verbindungen sowie Verfahren zu ihrer Herstellung und diese Verbindungen enthaltende Arzneimittel.

(30) Priorität: **04.07.87 DE 3722134**

(43) Veröffentlichungstag der Anmeldung:
**01.02.89 Patentblatt 89/05**

(45) Bekanntmachung des Hinweises auf die Patenterteilung:
**08.09.93 Patentblatt 93/36**

(84) Benannte Vertragsstaaten:
**BE CH DE FR GB IT LI NL**

(56) Entgegenhaltungen:
**EP-A- 0 000 074**
**EP-A- 0 062 199**
**EP-A- 0 103 833**
**EP-A- 0 212 551**
**DE-A- 2 428 792**
**DE-A- 2 658 558**

**CHEMICAL ABSTRACTS, Band 87, Nr. 25, 19. Dezember 1977, Seite 727, Abstract 201499q; U. HOERLEIN: "Nonsymmetric N-substituted bispidine (3,7-diazabicyclo[3.3.1]nonane)"**

**CHEMISCHE BERICHTE, Band 110, 1977, Seiten 3894-3899 ; U.HOERLEIN et al.: "Über unsymmetrisch N-substituierte Bispidine (3,7-Diazabicyclo (3.3.1)nonane), II"**

(73) Patentinhaber: **Kali-Chemie Pharma GmbH**
**Hans-Böckler-Allee 20**
**D-30173 Hannover(DE)**

(72) Erfinder: **Schön, Uwe**
**Föhrenkamp 12**
**D-3167 Burgdorf(DE)**
Erfinder: **Kehrbach, Wolfgang**
**Altenbekener Damm 41**
**D-3000 Hannover 1(DE)**
Erfinder: **Wolf, Klaus-Ullrich**
**Imkersweg 6**
**D-3162 Uetze-Hänigsen(DE)**

(74) Vertreter: **Lauer, Dieter, Dr.**
**c/o Kali-Chemie Aktiengesellschaft, Hans-Bockler-Allee 20**
**D-30173 Hannover (DE)**

EP 0 301 245 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3. 10/3.6/3.3. 1)

CHEMICAL ABSTRACTS, Band 88, Nr. 5, 30. Januar 1978, Seite 484, Abstract 37592n; W.N. SPECKAMP et al.: "Synthesis of functionally substituted 1-azaadamantanes. Anomalous 1,3-diol fragmentation"

CHEMICAL ABSTRACTS, Band 76, Nr. 25, 19. Juni 1972, Seite 408, Abstract 153122k; A.W.J.D. DEKKERS et al.: "Stereochemical spects in the mass spectra of N-tosyl-3-aza-7-carbethoxybycyclo[3.3.1]nonanes. Evidence for anchimeric assistance in the expulsion of a tosyl radical"

## Beschreibung

Die vorliegende Erfindung betrifft neue 3-Sulfonyl-3,7-diazabicyclo[3,3,1]nonan-Verbindungen und deren Salze sowie diese Verbindungen enthaltende pharmazeutische Zubereitungen und Verfahren zur Herstellung dieser Verbindungen.

Aus der DE-OS 26 58 558 sind 3-Alkanoyl- und 3-Aroyl-3,7-diazabicyclo[3,3,1]nonan-Derivate bekannt, für welche zentralanalgetische Wirkungen angegeben werden. Aus DE-OS 24 28 792 sind in 3- und 7-Stellung durch Alkyl- oder Phenylalkylreste substituierte 3,7-Diazabicyclo-[3,3,1]nonan-Derivate mit antiarrhythmischen Eigenschaften bekannt. In der EP-A-0 000 074 werden 7-Benzyl-3-phenylalkyl-3,7-diazabicyclo[3,3,1]nonan-Derivate mit ebenfalls antiarrhythmischen Wirkungen beschrieben. Weitere 3,7-Diazabicyclo[3,3,1]nonan-Derivate mit herzwirksamen, insbesondere antiarrhythmischen Eigenschaften sind aus der EP-A-0 103 833 bekannt. Außderdem werden ein 3,7-Diazabicyclo[3,3,1]nonan-Ringgerüst oder ähnliche Ringgerüste enthaltende Verbindungen auch beschrieben in EP-A 0 062 199, EP-A 0 212 551, Chem. Ber. 110, 1977, Seiten 3894 ff; Chem. Abs., 87, No 25, 1977, Seite 727, Abs. No 201499p; Chem. Abs., 88, No 5, 1978, Seite 484, Abs. No 3759n und Chem. Abs., 76 No 25, 1972, Seite 408, Abs. No 153122k.

Der vorliegenden Erfindung liegt die Aufgabe zugrunde, neue 3,7-Diazabicyclo[3,3,1]nonan-Verbindungen mit wertvollen pharmakologischen Eigenschaften zu entwickeln.

Es wurde nun gefunden, daß die neuen in 3-Stellung durch einen Sulfonylrest substituierten 3,7-Diazabicyclo[3,3,1]-nonan-Verbindungen wertvolle pharmakologische Eigenschaften besitzen und eine günstige pharmakologische Wirkung im gastrointestinalen Trakt entfalten. Sie zeichnen sich insbesondere durch eine regulierende Wirkung auf die Motilität des Magens aus.

Die vorliegende Erfindung betrifft daher neue 3-Sulfonyl-3,7-diazabicyclo[3,3,1]nonan-Verbindungen der allgemeinen Formel I

(s. Formel I)

worin

| | |
|---|---|
| $R^1$ | eine Alkylgruppe mit 1-6 Kohlenstoffatomen, eine Cycloalkylalkylgruppe mit 4-7 Kohlenstoffatomen oder Benzyl bedeutet, |
| $R^2$ | Wasserstoff oder Alkyl mit 1-4 Kohlenstoffatomen bedeutet und |
| $R^3$ | Wasserstoff oder Alkyl mit 1-4 Kohlenstoffatomen bedeutet oder |
| $R^2$ und $R^3$ | gemeinsam eine Alkylenkette mit 3-6 Kohlenstoffatomen bilden, und |
| $R^4$ | für Alkyl mit 1-4 Kohlenstoffatomen, für Thienyl, welches unsubstituiert oder durch Halogen substituiert ist, oder für eine $-(CH_2)_n-R^5$-Gruppe steht, worin n = 0-3 ist und |
| $R^5$ | eine Phenylgruppe bedeutet, welche unsubstituiert ist oder substituiert ist durch Alkyl mit 1-4 Kohlenstoffatomen, Alkoxy mit 1-4 Kohlenstoffatomen, Halogen, Trifluormethyl oder Nitro, |

sowie deren Säureadditionssalze.

Sofern in den Verbindungen der Formel I $R^1$ für eine Alkylgruppe steht, kann diese geradkettig oder verzweigt sein und 1 bis 6, vorzugsweise 2 bis 5 Kohlenstoffatome enthalten. Eine Cycloalkylalkylgruppe $R^1$ kann 4 bis 7 Kohlenstoffatome enthalten. Als besonders geeignete Reste $R^1$ haben sich geradkettige Alkylreste. z.B. der n-Butylrest, erwiesen.

Sofern die Substituenten $R^2$ und $R^3$ niederes Alkyl darstellen, können dieses Alkylgruppen geradkettig oder verzweigt sein und 1 bis 4, vorzugsweise 1 bis 3 Kohlenstoffatome enthalten und insbesondere Methyl bedeuten. Die Alkylgruppen $R^2$ und $R^3$ sind zweckmäßigerweise gleichartig, können jedoch auch verschieden sein. Sofern $R^2$ und $R^3$ gemeinsam eine Alkylenkette bilden, kann diese 3 bis 6, vorzugsweise 4 bis 5 Kohlenstoffatome enthalten.

Sofern $R^4$ eine niedere Alkylgruppe darstellt, kann diese geradkettig oder verzweigt sein und 1 bis 4 Kohlenstoffatome enthalten.

Sofern $R^4$ Thienyl darstellt, kann dieses unsubstituiert oder durch Halogen mono- oder disubstituiert sein. Als Halogensubstituenten eignen sich insbesondere Chlor oder Brom.

Vorzugsweise stellt $R^4$ eine $-(CH_2)_n-R^5$-Gruppe dar, worin n für 0 bis 3, vorzugsweise 0 steht. Die Phenylgruppe $R^5$ kann unsubstituiert sein oder mono-, di- oder trisubstituiert sein. Als Substituenten kommen die vorstehend genannten Substituenten in Frage. Niedere Alkyl- oder Alkoxy-Gruppen können geradkettig oder verzweigt sein und 1 bis 4 Kohlenstoffatome enthalten. Als Halogensubstituenten kommen Fluor, Chlor, Brom oder Jod in Frage. Bei Mehrfachsubstitution stellen die Substituenten bevorzugt Halogen, insbesondere Chlor, Brom oder auch Fluor, niederes Alkyl, Methoxy oder auch Nitro dar.

Erfindungsgemäß werden die neuen 3-Sulfonyl-3,7-diazabicyclo[3,3,1]nonan-Verbindungen der allgemeinen Formel I und deren Säureadditionssalze erhalten, indem man in an sich bekannter Weise Verbindungen der allgemeinen Formel II

(s. Formel II)

worin $R^1$, $R^2$ und $R^3$ obige Bedeutung besitzen, mit Sulfonsäurederivaten der allgemeinen Formel III

(s. Formel III)

worin $R^4$ obige Bedeutung besitzt und X eine reaktive Gruppe bedeutet, umsetzt und gewünschtenfalls freie Verbindungen der Formel I ih ihre Säureadditionssalze überführt oder die Säureadditionssalze in die freien Verbindungen der Formel I überführt.

Die Umsetzung der Sulfonsäurederivate der Formel III mit den 3,7-Diazabicyclo[3,3,1]nonan-Verbindungen der Formel II kann nach an sich zur Sulfonamidbildung durch Acylierung üblichen Methoden ausgeführt werden. Als reaktionsfähige Derivate kommen insbesondere Sulfonsäurehalogenide, vorzugsweise -chloride, und Anhydride der Sulfonsäuren $R^4$-$SO_2$-OH in Frage, z.B. Verbindungen der Formel III, worin die reaktive Gruppe X Halogen, insbesondere Chlor, oder eine Gruppe O-$SO_2R^4$, worin $R^4$ obige Bedeutung besitzt, bedeutet. Die Acylierung kann in einem unter den Reaktionsbedingungen inerten Lösungsmittel bei Temperaturen zwischen 0 °C und Siedetemperatur des Lösungsmittels erfolgen. Als Lösungsmittel eignen sich halogenierte Kohlenwasserstoffe wie Methylenchlorid oder Chloroform, aromatische Kohlenwasserstoffe wie Benzol, Toluol, Xylol oder Chlorbenzol, cyclische Äther wie Tetrahydrofuran oder Dioxan, Dimethylformamid oder Gemische dieser Lösungsmittel. Die Acylierung kann gewünschtenfalls in Gegenwart eines säurebindenden Mittels durchgeführt werden. Als säurebindende Mittel eignen sich anorganische Basen, insbesondere Alkalimetallcarbonate, oder organische Basen, insbesondere tert. Niederalkylamine und Pyridine, wie z.B. Triäthylamin oder 4-Dimethylaminopyridin.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden. Als pharmakologisch annehmbare Säureadditionssalze der Verbindungen der Formel I eignen sich beispielsweise deren Salze mit anorganischen Säuren, z.B. Halogenwasserstoffsäuren, insbesondere Chlorwasserstoffsäure, Schwefelsäure oder Phosphorsäuren, oder mit organischen Säuren, beispielsweise niederen aliphatischen Mono- oder Dicarbonsäuren wie Milchsäure, Maleinsäure, Fumarsäure oder Essigsäure, oder Sulfonsäuren, beispielsweise Niederalkylsulfonsäuren wie Methansulfonsäure oder gegebenenfalls im Benzolring durch Halogen oder niederes Alkyl substituierte Benzolsulfonsäuren wie p-Toluolsulfonsäure oder Cyclohexylaminosulfonsäure.

Für den Fall, daß $R^2$ und $R^3$ verschieden sind, können die Verbindungen in zwei stereoisomeren Formen auftreten. Die vorliegende Erfindung umfaßt sowohl die Isomerengemische wie auch die reinen Isomeren dieser Verbindungen der Formel I. Isomerengemische können auf an sich bekannte Weise auf der Stufe der Endverbindungen oder auf einer Zwischenproduktstufe in die einzelnen Isomeren aufgetrennt werden, beispielsweise durch fraktionierte Kristallisation oder durch säulenchromatographische Trennung.

Die als Ausgangsverbindungen verwendeten 3,7-Diazabicyclo[3,3,1]nonan-Verbindungen der Formel II sind aus den EP-A-0 000 074 und EP-A-0 103 833 und den DE-OS 24 28 792 und DE-OS 26 58 558 bekannt und/oder können nach den in diesen Schriften beschriebenen Methoden oder analog zu den in diesen Schriften beschriebenen Methoden auf an sich bekannte Weise hergestellt werden.

Beispielsweise können Verbindungen der Formel II erhalten werden, indem man aus Verbindungen der Formel IV

(s. Formel IV)

worin $R^1$, $R^2$ und $R^3$ obige Bedeutung besitzen und $R^6$ Benzyl bedeutet, die Benzylgruppe $R^6$ auf an sich bekannte Weise hydrogenolytisch abspaltet. Die hydrogenolytische Abspaltung der Gruppe $R^6$ kann mit Wasserstoff in Gegenwart eines Palladium/Kohle-Katalysators in einem organischen protischen, polaren Lösungsmittel, beispielsweise einem niederen Alkohol wie Äthanol, erfolgen. Die Hydrierung kann zweckmäßig bei Raumtemperatur und einem Wasserstoffdruck von ca. 5 bis 6 Atmosphären durchgeführt werden.

Ausgangsverbindungen der Formel IVa

(s. Formel IVa)

worin $R^1$ und $R^6$ obige Bedeutung besitzen, können in an sich bekannter Weise erhalten werden, indem man Piperidonverbindungen der allgemeinen Formel V

(s. Formel V)

worin $R^1$ obige Bedeutung besitzt, mit Aminverbindungen der allgemeinen Formel VI

(s. Formel VI)

worin $R^6$ obige Bedeutung besitzt, und 2 Mol Formaldehyd in einer Mannich-Reaktion zu Keto-Verbindungen der allgemeinen Formel VII

(s. Formel VII)

worin $R^1$ und $R^6$ obige Bedeutung besitzen, umsetzt und diese anschließend reduziert. Die Umsetzung der Amine der Formel VI mit Formaldehyd und den Piperidonen der Formel V kann unter für Mannich-Kondensationen üblichen Bedingungen erfolgen. Zum Beispiel kann die Umsetzung des Amins mit dem Piperidon und Formaldehyd, gewünschtenfalls in Form von Paraformaldehyd, in einem organischen Lösungsmittel, beispielsweise einem cyclischen Äther, einem halogenierten Kohlenwasserstoff oder vorzugsweise einem niederen Alkohol, in Gegenwart einer das Amin neutralisierenden Menge einer Säure, beispielsweise Eisessig oder Salzsäure erfolgen. Zweckmäßig wird die Reaktion bei Raumtemperatur durchgeführt. Die Reduktion der entstandenen Keto-Verbindungen der Formel VII kann nach an sich zur Reduktion von Ketonen üblichen Methoden durchgeführt werden. Insbesondere eignet sich die Reduktion nach Wolff-Kishner mittels Hydrazin. Beispielsweise kann die Umsetzung mit Hydrazin in Gegenwart von Alkalimetallhydroxid in einem hochsiedenden Lösungsmittel, beispielsweise Triäthylenglycol, bei Siedetemperatur des Lösungsmittels erfolgen.

Verbindungen der Formel IVb

(s. Formel IVb)

worin $R^1$ und $R^6$ obige Bedeutung besitzen und $R^7$ niederes Alkyl bedeutet, können aus den Verbindungen der Formel VII hergestellt werden, indem man diese in an sich bekannter Weise zunächst mit einem Alkylhalogenid in einer Grignard-Reaktion in die entsprechenden Carbinole überführt und anschließend auf an sich bekannte Weise die alkoholische Hydroxygruppe in eine abspaltbare Fluchtgruppe, z.B. eine Sulfonyloxygruppe, beispielsweise die Tosyloxygruppe, überführt und diese auf an sich bekannte Weise reduktiv abspaltet.

Verbindungen der Formel IVc

(s. Formel IVc)

worin $R^{2'}$ und $R^{3'}$ die für $R^2$ und $R^3$ angegebenen Bedeutungen mit Ausnahme von Wasserstoff besitzen und $R^1$, und $R^6$ obige Bedeutung besitzen, können beispielsweise ausgehend von Verbindungen der Formel VIII

(s. Formel VIII)

worin $R^1$, $R^{2'}$ und $R^{3'}$ obige Bedeutung besitzen, erhalten werden. Hierzu werden die Tetraoxo-Verbindungen der Formel VIII zunächst mit Benzylhalogeniden der Formel IX

(s. Formel IX)

worin $R^6$ obige Bedeutung besitzt und Hal für Halogen, insbesondere Chlor oder Brom, steht, zu den N,N′-disubstituierten Tetraoxo-Verbindungen der Formel X

(s. Formel X)

worin $R^1$, $R^{2'}$, $R^{3'}$ und $R^6$ obige Bedeutung besitzen, umgesetzt und diese anschließend zu den Verbindungen der Formel IVc reduziert. Die Umsetzung der Diimid-Verbindungen der Formel VIII mit den Verbindungen der Formel IX kann nach an sich zur Alkylierung von Imiden üblichen Methoden erfolgen.

Die Umsetzung findet zweckmäßigerweise in einem unter den Reaktionsbedingungen inerten Lösungsmittel in Gegenwart einer Base bei erhöhter Temperatur, beispielsweise Siedetemperatur des Lösungsmittels, statt. So eignen sich beispeilsweise Alkalimetallcarbonate oder -hydride in Dimethylformamid oder Alkalimetallalkoholate in einem niederen Alkohol. Zweckmäßigerweise wird das Benzylhalogenid im überschuß eingesetzt.

Die 2,4,6,8-Tetraoxo-3,7-diazabicyclo[3,3,1]nonan-Verbindungen der Formel VIII sind bekannt und/oder können nach der von Hörlein beschriebenen Methode (Eur. J. Med. Chem. 12, 301-305) hergestellt werden durch Ringschluß von 2,6-Dioxo-3,5-dicyanpiperidin-Verbindungen der Formel XI

(s. Formel XI)

worin $R^1$, $R^{2'}$ und $R^{3'}$ obige Bedeutung besitzen, in hochprozentigen Säure-Wasser-Gemischen. Die 2,6-Dioxo-3,5-dicyanpiperidine XI werden ihrerseits auf bekannte Weise erhalten durch Kondensation von entsprechend substituierten Alkylidencyanessigestern der Formel XII

(s. Formel XII)

worin $R^{2'}$ und $R^{3'}$ obige Bedeutung besitzen, mit Cyanacetamiden der Formel XIII

(s. Formel XIII)

worin $R^1$ obige Bedeutung besitzt.

Die Verbindungen der Formel I und ihre pharmakologisch akzeptablen Säureadditionssalze besitzen interessante pharmakologische Eigenschaften und zeichnen sich insbesondere durch eine günstige Wirkung auf die Motilität des Magens aus. So werden im Tierexperiment unter dem Einfluß von Verbindungen der Formel I die peristaltischen Wellen das Magens verstärkt, wobei sich die Frequenz der Bewegungen nicht signifikant verändert.

Beschreibung der pharmakologischen Untersuchungsmethoden:

1. Bestimmung der minimalen toxischen Dosis.

Männlichen Mäusen von 20-25 g Gewicht werden i.p. Maximaldosen von 200 mg/kg der Testsubstanz verabreicht. Die Tiere werden 3 Stunden lang sorgfältig auf Toxizitätssymptome beobachtet. über einen Zeitraum von 24 Stunden nach der Applikation werden zusätzlich alle Symptome und Todesfälle registriert. Begleitsymptome werden ebenfalls beobachtet und registriert. Wenn Tod oder starke toxische Symptome beobachtet werden, werden weiteren Mäusen zunehmend geringere Dosen verabreicht, bis keine toxischen Symptome mehr auftreten. Die niedrigste Dosis, welche Tod oder starke toxische Symptome hervorruft, wird als minimale toxische Dosis angegeben.

2. Bestimmung der Wirkung auf die Motilität des Magens in narkotisierten Ratten.

Nüchterne Ratten des Stammes SIV 50 mit einem Körpergewicht von 180-250 g werden mit einer Ketamin/Xylazin-Mischung anästhesiert. Die Tiere werden tracheotomiert und laparotomiert. Nach Anlegen einer Pylorusligatur wird eine Magensonde in den Magen eingeführt und am anderen Ende über einen Drei-Wege-Hahn mit einem geeichten Druckaufnehmer (Statham-Element P 23 ID) verbunden. Anschließend wird der Magen der Tiere über die Sonde mit 2-3 ml Wasser gefüllt. Die Druckschwankungen im Magen werden vor und nach Applikation der Testsubstanzen mit Hilfe eines Watanabe-Multicorders (MC 641) aufgezeichnet. Die Differenzen zwischen den mittleren Amplituden vor und nach der Behandlung werden bestimmt und die durch die Testsubstanzen bewirkte Änderung der mittleren Amplituden werden bezogen auf die vor der Behandlung erhaltenen Werte angegeben.

In der vorstehenden Testmothede zeigen die Verbindungen nach i.p.-Applikation von 100 µMol/kg beispielsweise eine Amplitudensteigerung um die in der folgenden Tabelle P angegebenen Faktoren. Die in Tabelle P angegebenen Beispielsnummern beziehen sich auf die nachstehenden Herstellungsbeispiele.

Tabelle P

| Testsubstanz Beispiel Nr. | Magenmotilitätsfördernde Wirkung in der Ratte Faktor der Amplitudensteigerung bei Dosis von 100 $\mu$Mol/kg i.p. | Minimale toxische Dosis mg/kg Maus i.p. |
|---|---|---|
| 1 | > 10 | 200 |
| 2 | > 10 | > 200 |
| 3 | 8,8 | 200 |
| 4 | > 10 | 100 |
| 6 | 6,7 | 200 |
| 7 | 5,7 | > 200 |
| 8 | > 10 | 100 |
| 9 | > 10 | 200 |
| 10 | > 10 | 200 |
| 17 | 5,0 | > 200 |
| 18 | 7,3 | 200 |
| 23 | > 10 | 200 |
| 26 | > 10 | 100 |
| 27 | > 10 | 200 |
| 29 | 9,4 | > 200 |
| 31 | 3,5 | 200 |
| 32 | 1,9 | 200 |
| 34 | 7,9 | 200 |
| 36 | 4,6 | 200 |
| 37 | 4,2 | > 200 |
| 41 | 8,8 | 200 |
| 43 | > 10 | - |
| 48 | 4,8 | > 200 |
| 54 | 10 | > 200 |
| 56 | > 10 | 100 |
| 58 | 9,3 | 200 |
| 61 | 4,2 | > 200 |
| 62 | 3,0 | > 200 |
| 66 | 4,7 | > 200 |

Wie aus den vorstehenden Daten ersichtlich, bewirken die erfindungsgemäßen Verbindungen einen beträchtlichen Anstieg der mittleren Amplitude der peristaltischen Druckwellen des Magens.

Aufgrund dieser Wirkungen sind die Verbindungen der Formel I und ihre physiologisch verträglichen Säureadditionssalze in der Gastroenterologie als Arzneimittel für größere Säugetiere, insbesondere Menschen, zur Prophylaxe und Behandlung von Motilitätsstörungen im Magen-Darm-Trakt geeignet. So sind die

Substanzen beispielsweise nützlich zur Behandlung von verschiedenen durch Motilitätsstörungen des Magen-Darm-Traktes hervorgerufenen Beschwerden wie übelkeit, Völlegefühl oder Oberbauchschmerzen.

Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoff enthalten als orale Präparate. Im allgemeinen eignen sich jedoch für Applikationen an größeren Säugetieren, insbesondere Menschen, Arzneiformen mit einem Wirkstoffgehalt von 5-50 mg pro Einzeldosis.

Als Heilmittel können die Verbindungen der Formel I und ihre physiologisch verträglichen Säureadditionssalze mit üblichen pharmazeutischen Hilfsstoffen in galenischen Zubereitungen wie z.B. Tabletten, Kapseln, Suppositorien oder Lösungen enthalten sein. Diese galenischen Zubereitungen können nach an sich bekannten Methoden hergestellt werden unter Verwendung üblicher fester Trägerstoffe wie z.B. Milchzucker, Stärke oder Talkum oder flüssiger Verdünnungsmittel wie z.B. Wasser, fetten Ölen oder flüssigen Paraffinen und unter Verwendung von pharmazeutisch üblichen Hilfsstoffen, beispielsweise Tablettensprengmitteln, Lösungsvermittlern oder Konservierungsmitteln.

Die nachfolgenden Beispiele sollen die Erfindung näher erläutern, jedoch deren Umfang in keiner Weise beschränken.

Beispiel 1:

7-(n-Butyl)-3-[(3,4-dichlorphenyl)-sulfonyl]-9,9-dimethyl-3,7-diazabicyclo[3,3,1]nonan.

Zu einer Lösung von 2,4 g (= 0,0114 Mol) 7-(n-Butyl)-9,9-dimethyl-3,7-diazabicyclo[3,3,1]nonan (= Substanz Nr. C1 der nachfolgenden Tabelle C) in 40 ml Dichlormethan wird unter Eiskühlung eine Lösung von 2,8 g (= 0,0114 Mol) 3,4-Dichlorbenzolsulfonsäurechlorid in 20 ml Dichlormethan zugetropft. Anschließend wird das Eisbad entfernt und das Reaktionsgemisch 3 Stunden bei Raumtemperatur weitergerührt. Hierbei fällt das Hydrochlorid der Titelverbindung als weißer Niederschlag aus. Zur Vervollständigung der Kristallisation wird das Reaktionsgemisch weitere 12 Stunden im Kühlschrank stehengelassen. Sodann werden die Kristalle abgesaugt und bei 60 °C im Vakuumtrockenschrank getrocknet. Es werden 3,3 g 7-(n-Butyl)-3-[(3,4-dichlorphenyl)-sulfonyl]-9,9-dimethyl-3,7-diazabicyclo[3,3,1]nonan-hydrochlorid mit einem Schmelzpunkt von 170-173 °C erhalten.

Beispiel 2:

7-Benzyl-3-[(thien-2-yl)-sulfonyl]-9,9-dimethyl-3,7-diazabicyclo[3,3,1]nonan.

A) Zu einer Lösung von 3,5 g (= 0,014 Mol) 7-Benzyl-9,9-dimethyl-3,7-diazabicyclo[3,3,1]nonan (= Substanz Nr. C8 der nachfolgenden Tabelle C) in 40 ml Dichlormethan wird unter Eiskühlung eine Lösung von 2,87 g (= 0,015 Mol) Thiophen-2-sulfonylchlorid in 20 ml Dichlormethan zugetropft. Anschließend wird das Eisbad entfernt und das Reaktionsgemisch 3 Stunden bei Raumtemperatur weitergerührt. Sodann wird das Reaktionsgemisch durch Zugabe von wäßriger Natriumhydroxidlösung alkalisiert, die wäßrige Phase abgetrennt und zweimal mit Dichlormethan extrahiert. Die vereinigten Dichlormethanphasen werden über Magnesiumsulfat getrocknet und eingeengt. Hierbei werden 4,0 g der Titelverbindung (Base) als Öl erhalten.

B) 4.0 g der vorstehend erhaltenen öligen Base werden in 40 ml Essigsäureäthylester gelöst, und die Lösung wird unter Eiskühlung mit einer Lösung von 1,54 g Weinsäure in 40 ml Aceton versetzt. Hierbei fällt das Hydrogentartrat der Titelverbindung kristallin aus. Die Kristalle werden abgesaugt und im Vakuumtrockenschrank bei 50 °C getrocknet. Es werden 4,3 g 7-Benzyl-3-[(thien-2-yl)sulfonyl]-9,9-dimethyl-3,7-diazabicyclo[3,3,1]nonan-hydrogentartrat mit einem Schmelzpunkt von 148-153 °C erhalten.

Analog zu den in den vorstehenden Beispielen 1 und 2 bechriebenen Verfahren können auch die in der nachstehenden Tabelle 1 angeführten Verbindungen der Formel I ausgehend von entsprechenden Verbindungen der Formel II erhalten werden.

TABELLE 1

| Beispiel Nr. | $R^1$ | $R^2$ | $R^3$ | $R^4$ | Bemerkungen Fp. in °C |
|---|---|---|---|---|---|
| 3 | n-$C_4H_9$ | $CH_3$ | $CH_3$ | phen-$(CH_2)_2$- | 1,4 Tart: 78 |
| 4 | n-$C_4H_9$ | $CH_3$ | $CH_3$ | phen | B: öl, Tart: am |
| 5 | n-$C_4H_9$ | $CH_3$ | $CH_3$ | 4-$CH_3$-phen | B: 85 |
| 6 | n-$C_4H_9$ | $CH_3$ | $CH_3$ | 2,5-di-$CH_3$-phen | 1,5 Tart: 85 |
| 7 | n-$C_4H_9$ | $CH_3$ | $CH_3$ | 2,4,6-tri-isoprop-phen | B: öl, Tart: am |
| 8 | n-$C_4H_9$ | $CH_3$ | $CH_3$ | 4-(n-$C_4H_9$)-phen | B: öl, Tart: am |
| 9 | n-$C_4H_9$ | $CH_3$ | $CH_3$ | 4-$CH_3$O-phen | B: öl, Tart: am |
| 10 | n-$C_4H_9$ | $CH_3$ | $CH_3$ | 2,5-di-$CH_3$O-phen | B: 72-73 |
| 11 | n-$C_4H_9$ | $CH_3$ | $CH_3$ | 3,4-di-$CH_3$O-phen | B: 94 |
| 12 | n-$C_4H_9$ | $CH_3$ | $CH_3$ | 2-Cl-phen | B: öl, Tart:am |
| 13 | n-$C_4H_9$ | $CH_3$ | $CH_3$ | 4-Cl-phen | HCl: 132-137 |
| 14 | n-$C_4H_9$ | $CH_3$ | $CH_3$ | 3-Cl-phen | Tart: 102 |
| 15 | n-$C_4H_9$ | $CH_3$ | $CH_3$ | 2,5-di-Cl-phen | B: öl, Tart: am |

B = Base, öl = ölig, phen = Phenyl, isoprop = Isopropyl, thien = Thienyl, cyclohex = Cyclohexyl, Tart = Hydrogentartrat, HCl = Hydrochlorid, am = amorph

| Beispiel Nr. | $R^1$ | $R^2$ | $R^3$ | $R^4$ | Bemerkungen Fp. in °C |
|---|---|---|---|---|---|
| 16 | n-$C_4H_9$ | $CH_3$ | $CH_3$ | $CH_3$ | B: öl |
| 17 | n-$C_4H_9$ | $CH_3$ | $CH_3$ | 3,5-di-Cl-phen | 0,8 HCl: 194 |
| 18 | $C_2H_5$ | $CH_3$ | $CH_3$ | 3-Cl-phen | 1,4 Tart: 100 |
| 19 | n-$C_5H_{11}$ | $CH_3$ | $CH_3$ | 3-Cl-phen | Tart: 159-160 |
| 20 | n-$C_4H_9$ | $CH_3$ | $CH_3$ | 4-F-phen | B: öl, Tart: am |
| 21 | n-$C_4H_9$ | $CH_3$ | $CH_3$ | 4-F-3-Cl-phen | HCl: 165 |
| 22 | n-$C_4H_9$ | $CH_3$ | $CH_3$ | 4-Br-phen | HCl: 177-178 |
| 23 | n-$C_5H_{11}$ | $CH_3$ | $CH_3$ | 4-Br-phen | HCl: 218-222 |
| 24 | n-$C_4H_9$ | $CH_3$ | $CH_3$ | 4-J-phen | HCl: 222-227 |
| 25 | n-$C_4H_9$ | $CH_3$ | $CH_3$ | 2-$CF_3$-phen | B: 63 |
| 26 | n-$C_4H_9$ | $CH_3$ | $CH_3$ | 3-$CF_3$-phen | HCl: 114 |
| 27 | n-$C_4H_9$ | $CH_3$ | $CH_3$ | 4-$NO_2$-phen | 1,55 HCl: 79 |
| 28 | n-$C_4H_9$ | $CH_3$ | $CH_3$ | 2-$NO_2$-phen | Tart: 173 |
| 29 | n-$C_4H_9$ | $CH_3$ | $CH_3$ | 3-$NO_2$-4-Cl-phen | B: 73-75 |
| 30 | n-$C_4H_9$ | n-$C_3H_7$ | n-$C_3H_7$ | 4-Br-phen | HCl: 157-158 |
| 31 | n-$C_4H_9$ | $CH_3$ | $CH_3$ | 2-$CH_3$-6-Cl-phen | B: öl, Tart: am |
| 32 | n-$C_4H_9$ | $CH_3$ | $CH_3$ | 2-$CH_3O$-5-Br-phen | B: 65-68 |
| 33 | n-$C_4H_9$ | $CH_3$ | $CH_3$ | 2-thien | HCl: 213 |
| 34 | $C_2H_5$ | $CH_3$ | $CH_3$ | 2-thien | B: 94-96 |
| 35 | n-$C_5H_{11}$ | $CH_3$ | $CH_3$ | 2-thien | B: öl, Tart: am |

| Beispiel Nr. | $R^1$ | $R^2$ | $R^3$ | $R^4$ | Bemerkungen Fp. in °C |
|---|---|---|---|---|---|
| 36 | n-$C_4H_9$ | $CH_3$ | $CH_3$ | 5-Cl-2-thien | B: öl, Tart: am |
| 37 | n-$C_4H_9$ | $CH_3$ | $CH_3$ | 4,5-di-Br-2-thien | B: 68 |
| 38 | n-$C_4H_9$ | $CH_3$ | $CH_3$ | 2,5-di-Cl-3-thien | 1,25 Tart: 84-88 |
| 39 | $(CH_3)_2CH$- | $CH_3$ | $CH_3$ | 4-Br-phen | B: 140 |
| 40 | $(CH_3)_2CH$- | $CH_3$ | $CH_3$ | 3-Cl-phen | B: 130 |
| 41 | $(CH_3)_2CH$- | $CH_3$ | $CH_3$ | 2-thien | B: 98 |
| 42 | Cyclohex-$CH_2$- | $CH_3$ | $CH_3$ | 4-Br-phen | B: öl, Tart: am |
| 43 | Cyclohex-$CH_2$- | $CH_3$ | $CH_3$ | 3-Cl-phen | B: öl, Tart: am |
| 44 | Cyclohex-$CH_2$- | $CH_3$ | $CH_3$ | 2-thien | B: öl, Tart: am |
| 45 | $CH_3$ | H | H | 4-$CH_3$-phen | B: 97 |
| 46 | n-$C_4H_9$ | H | H | 4-$CH_3$-phen | B: 55 |
| 47 | n-$C_4H_9$ | H | H | 4-Br-phen | B: 165-166 |
| 48 | $CH_3$ | H | H | 4-Br-phen | B: 196 |
| 49 | n-$C_4H_9$ | H | H | 2-thien | 1,3 Tart: 113 |
| 50 | phen-$CH_2$- | H | H | 4-$CH_3$-phen | B: 132 |
| 51 | phen-$CH_2$- | H | H | 3-Cl-phen | B: 159 |
| 52 | phen-$CH_2$- | H | H | 2-thien | Tart: 100 |
| 53 | phen-$CH_2$- | $CH_3$ | $CH_3$ | 4-Br-phen | Tart: 149-150 |
| 54 | phen-$CH_2$- | $CH_3$ | $CH_3$ | 4-F-phen | Tart: 99-100 |
| 55 | phen-$CH_2$- | $CH_3$ | $CH_3$ | 3-Cl-phen | B: öl, Tart: am |

| Beispiel Nr. | $R^1$ | $R^2$ | $R^3$ | $R^4$ | Bemerkungen Fp. in °C |
|---|---|---|---|---|---|
| 56 | $n-C_4H_9$ | $CH_3$ | $CH_3$ | phen-$CH_2$- | B: 113 |
| 57 | $n-C_4H_9$ | $-(CH_2)_5-$ | | 4-Br-phen | HCl: 211 |
| 58 | $n-C_4H_9$ | $-(CH_2)_5-$ | | 3-Cl-phen | Tart: 202 |
| 59 | $n-C_4H_9$ | $-(CH_2)_5-$ | | 2-thien | B: 97 |
| 60 | $(CH_3)_2CH-CH_2-$ | $-(CH_2)_5-$ | | 4-Br-phen | B: 135 |
| 61 | $(CH_3)_2CH-CH_2-$ | $-(CH_2)_5-$ | | 3-Cl-phen | B: 134-135 |
| 62 | Cyclohex-$CH_2$- | $-(CH_2)_5-$ | | 4-Br-phen | B: 143-145 |
| 63 | Cyclohex-$CH_2$- | $-(CH_2)_5-$ | | 2-thien | B: 112 |
| 64 | $n-C_4H_9$ | $n-C_3H_7$ | $n-C_3H_7$ | 2-thien | B: 83 |
| 65 | $n-C_4H_9$ | $n-C_3H_7$ | $n-C_3H_7$ | 3,4-di-Cl-phen | B: 96 |
| 66 | $n-C_4H_9$ | $n-C_3H_7$ | $n-C_3H_7$ | 3-Cl-phen | B: 45-48, HCl: 124-126 |

Die verwendeten Ausgangsstoffe wurden nach den folgenden allgemeinen Arbeitsvorschriften hergestellt:

A) Allgemeine Arbeitsvorschrift zur Herstellung von 3,7-disubstituierten 2,4,6,8-Tetraoxo-3,7-diazabicyclo-[3,3,1]nonan-Verbindungen der Formel X durch Umsetzung von 2,4,6,8-Tetraoxo-3,7-diazabicyclo[3,3,1]-nonan-Verbindungen der Formel VIII mit Benzylhalogeniden der Formel IX.

a) Umsetzung von N-monosubstituierten Verbindungen der Formel VIII, worin $R^1$ nicht = H.

Eine Mischung aus 0,1 Mol der Imid-Verbindung der Formel VIII, 0,2 Mol Kaliumcarbonat und 0,15 Mol Benzylhalogenid der Formel IX in 390 ml Dimethylformamid wird 3 bis 7 Stunden lang am Rückfluß erhitzt. Anschließend wird von dem gebildeten Niederschlag anorganischer Salze abfiltriert und die klare Lösung bis zur Trockne eingeengt. Der verbleibende Rückstand wird in Wasser und Essigester gelöst. Die organische Lösung wird abgetrennt, zweimal mit Wasser gewaschen, über Magnesiumsulfat getrocknet, filtriert und eingeengt. Falls die gebildete Tetraoxo-Verbindung der Formel X hierbei bereits kristallin anfällt, genügt zur weiteren Reinigung eine einfach Umkristallisierung. Anderenfalls kann es notwendig sein, daß erhaltene Rohprodukt säulenchromatographisch über Kieselgel oder Aluminiumoxid unter Verwendung von Essigester-Hexan-Gemischen als Elutionsmittel zu reinigen.

b) Umsetzung solcher Verbindungen der Formel VIII, worin $R^1$ = H.

Zur Disubstitution der Verbindungen der Formel VIII, worin $R^1$ = H durch die Benzylhalogenide der Formel IX wird die vorstehende allgemeine Arbeitsvorschrift zur Monosubstituierung der Verbindungen der Formel VIII, worin $R^1$ nicht = H, abgewandelt. Anstelle der vorstehend angegebenen Reaktionsmischung wird eine Mischung aus 0,1 Mol der Tetraoxoverbindung der Formel VIII, 0,25 Mol Kaliumcarbonat und 0,3 Mol Benzylhalogenid der Formel IX in 300 ml Dimethylformamid eingesetzt.

Nach den vorstehenden allgemeinen Arbeitsvorschriften wurden die folgenden in Tabelle A angegebenen Verbindungen hergestellt.

## Tabelle A

Verbindungen der Formel X

| Substanz Nr. | $R^1$ | $R^{2'}$ | $R^{3'}$ | $R^6$ | Bemerkungen Fp in °C |
|---|---|---|---|---|---|
| A1 | $n-C_4H_9$ | $CH_3$ | $CH_3$ | Benz | Fp: 110 |
| A2 | $C_2H_5$ | $CH_3$ | $CH_3$ | Benz | öl* |
| A3 | $n-C_5H_{11}$ | $CH_3$ | $CH_3$ | Benz | Fp: 85- 86 |
| A4 | $n-C_4H_9$ | $-(CH_2)_5-$ | | Benz | Fp: 100 |
| A5 | $n-C_4H_9$ | $n-C_3H_7$ | $n-C_3H_7$ | Benz | öl* |
| A6 | $(CH_3)_2CH-$ | $CH_3$ | $CH_3$ | Benz | Fp: 170-174 |
| A7 | Cyclohex-me | $CH_3$ | $CH_3$ | Benz | Fp: 129-131 |
| A8 | Benz | $CH_3$ | $CH_3$ | Benz | Fp: 155-157 |
| A9 | $(CH_3)_2CH-CH_2-$ | $-(CH_2)_5$ | | Benz | Fp: 126-128 |
| A10 | Cyclohex-me | $-(CH_2)_5-$ | | Benz | Fp: 137 |

Benz = Benzyl, Cyclohex-me = Cyclohexylmethyl,
*öl = wurde als öl weiterverarbeitet

B) Allgemeine Arbeitsvorschrift zur Reduktion von 2,4,6,8-Tetraoxo-3,7-diazabicyclo[3,3,1]nonan-Verbindungen der Formel X zu 3,7-Diazabicyclo[3,3,1]nonan-Verbindungen der Formel IVc.

In einem Dreihalskolben werden 0,1 Mol Lithiumaluminiumhydrid in 100 ml einer Lösung aus 70 ml absolutem Tetrahydrofuran und 30 ml absolutem Toluol auf eine Ölbadtemperatur von 80 °C erwärmt.

Sodann werden langsam 0,025 Mol der Tetraoxoverbindung in 100 ml einer Mischung Tetrahydrofuran/Toluol 70/30 zugetropft. Das Reaktionsgesmich wird 2 bis 4 Stunden lang bei 120 °C reagieren gelassen. Anschließend wird unter basischen Bedingungen hydrolysiert und mit Methylenchlorid extrahiert. Die organische Phase wird abgetrennt, über Magnesiumsulfat getrocknet und eingeengt. Die gebildeten 3,7-Diazabicyclo[3,3,1]nonan-Verbindungen werden durch Kugelrohrdestillation unter vermindertem Druck abgetrennt.

Nach dieser allgemeinen Arbeitsvorschrift zur Reduktion mittels Lithiumaluminiumhydrid wurden die in der nachstehenden Tabelle B angegebenen 3,7-Diazabicyclo[3,3,1]nonan-Verbindungen der Formel IVc hergestellt.

## Tabelle B

Verbindungen der Formel IVc

| Substanz IVc Nr. | $R^1$ | $R^2{}'$ | $R^3{}'$ | $R^6$ | Bemerkungen Siedepunkt in °C (0,01Torr) Fp in °C |
|---|---|---|---|---|---|
| B1 | $n-C_4H_9$ | $CH_3$ | $CH_3$ | Benz | Sdp: 170 |
| B2 | $C_2H_5$ | $CH_3$ | $CH_3$ | Benz | Sdp: 185-190 |
| B3 | $n-C_5H_{11}$ | $CH_3$ | $CH_3$ | Benz | Sdp: 160-170 |
| B4 | $n-C_4H_9$ | $-(CH_2)_5-$ | | Benz | Sdp: 220 |
| B5 | $n-C_4H_9$ | $n-C_3H_7$ | $n-C_3H_7$ | Benz | Sdp: 200 |
| B6 | $(CH_3)_2CH-$ | $CH_3$ | $CH_3$ | Benz | Sdp: 150 |
| B7 | Cyclohex-me | $CH_3$ | $CH_3$ | Benz | Sdp: 170 |
| B8 | Benz | $CH_3$ | $CH_3$ | Benz | Sdp: 230 |
| B9 | $(CH_3)_2CH-CH_2-$ | $-(CH_2)_5$ | | Benz | Sdp: n.b. |
| B10 | Cyclohex-me | $-(CH_2)_5-$ | | Benz | Fp: 95 |

Benz = Benzyl, Cyclohex-me = Cyclohexylmethyl
n.b. = nicht bestimmt, ohne Reinigung weiterverarbeitet
geengt. Die gebildeten 3,7-Diazabicyclo[3,3,1]nonan-Verbindungen werden durch Kugelrohrdestillation unter vermindertem Druck abgetrennt.

C) Allgemeine Arbeitsvorschrift zur Debenzylierung der 3,7-disubstituierten 3,7-Diazabicyclo[3,3,1]nonan-Verbindungen der Formel IV zu N-monosubstituierten 3,7-Diazabicyclo[3,3,1]nonan-Verbindungen der Formel II.

0,2 Mol einer Verbindung der Formel IV werden in 600 ml Äthanol gelöst und die Lösung wird mit 10 g Palladium/Kohle-Katalysator versetzt. Das Reaktionsgemsich wird bei Raumtemperatur unter einem Wasserstoffdruck von 5 Atmosphären ca. 6 Stunden hydriert. Nach Beendigung der Hydrierung wird die Lösung von dem Katalysator abgetrennt und eingeengt. Die gebildeten Verbindungen der Formel II

werden mit Hilfe von Kugelrohrdestillation unter vermindertem Druck abgetrennt.

Nach der vorstehenden allgemeinen Vorschrift zur Debenzylierung wurden die in der folgenden Tabelle C angegebenen 3,7-Diazabicylo[3,3,1]nonan-Verbindungen der Formel II hergestellt.

## Tabelle C

Verbindungen der Formel II

| Sub-stanz Nr. | $R^1$ | $R^2$ | $R^3$ | Bemerkungen Siedepunkt in °C (0,01 Torr) |
|---|---|---|---|---|
| C1 | $n-C_4H_9$ | $CH_3$ | $CH_3$ | Sdp: 145 |
| C2 | $C_2H_5$ | $CH_3$ | $CH_3$ | Sdp: 131 |
| C3 | $n-C_5H_{11}$ | $CH_3$ | $CH_3$ | Sdp: 150 |
| C4 | $n-C_4H_9$ | $-(CH_2)_5-$ | | n.b. |
| C5 | $n-C_4H_9$ | $n-C_3H_7$ | $n-C_3H_7$ | Sdp: 220 (1,5 Torr) |
| C6 | $(CH_3)_2CH-$ | $CH_3$ | $CH_3$ | Sdp: 150 |
| C7 | Cyclohex-me | $CH_3$ | $CH_3$ | n.b. |
| C8 | Benz | $CH_3$ | $CH_3$ | Sdp: 180 |
| C9 | $(CH_3)_2CH-CH_2-$ | $-(CH_2)_5-$ | | Sdp: 180 |
| C10 | Cyclohex-me | $-(CH_2)_5-$ | | Sdp: 220 |

Benz = Benzyl, Cyclohex-me = Cyclohexylmethyl

n.b. = nicht bestimmt, ohne Reinigung weiterverarbeitet

Beispiel I:

7-(n-Butyl)-3-[(3,4-dichlorphenyl)-sulfonyl]-9,9-dimethyl-3,7-diazabicyclo[3,3,1]nonan-hydrchlorid enthaltende Tabletten.

Es werden Tabletten in folgender Zusammensetzung pro Tablette hergestellt:

| | |
|---|---|
| 7-(n-Butyl)-3-[(3,4-dichlorphenyl)-sulfonyl]-9,9-dimethyl-3,7-diazabicyclo[3,3,1]nonan-hydrochlorid | 20 mg |
| Maisstärke | 60 mg |
| Milchzucker | 135 mg |
| Gelatine (als 10%-ige Lösung) | 6 mg |

Der Wirkstoff, die Maisstärke und der Milchzucker werden mit der 10 %-igen Gelatine-Lösung eingedickt. Die Paste wird zerkleinert und das entstandene Granulat wird auf ein geeignetes Blech gebracht und getrocknet. Das getrocknete Granulat wird durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

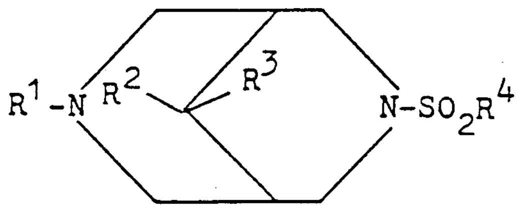

(I)

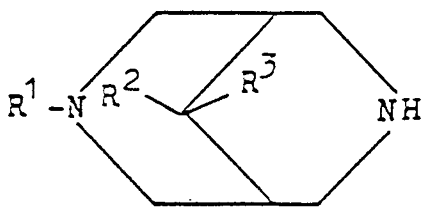

(II)

$R^4$-$SO_2$-X (III)

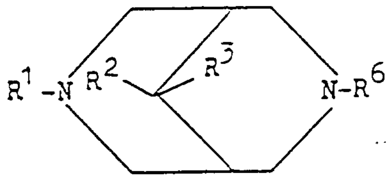

(IV)

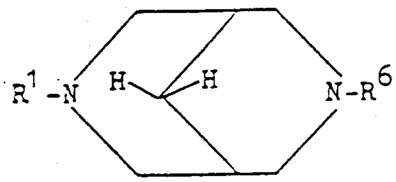

(IVa)

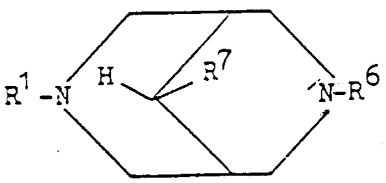

(IVb)

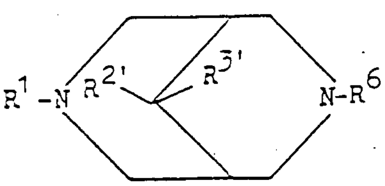

(IVc)

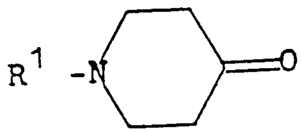

(V)

$NH_2$-$R^6$ (VI)

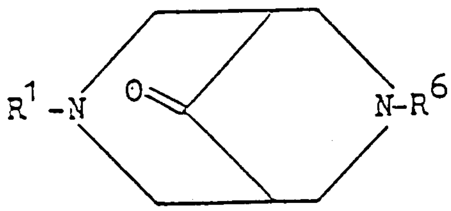

(VII)

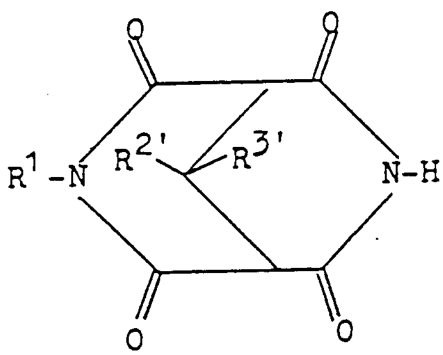

(VIII)

$R^6$-Hal (IX)

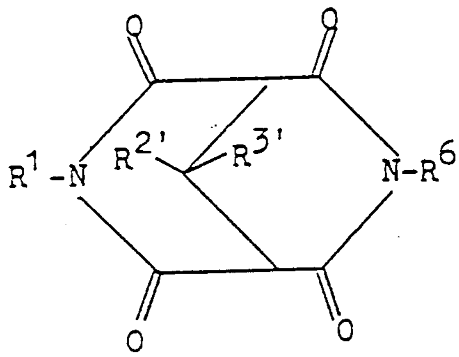

(X)

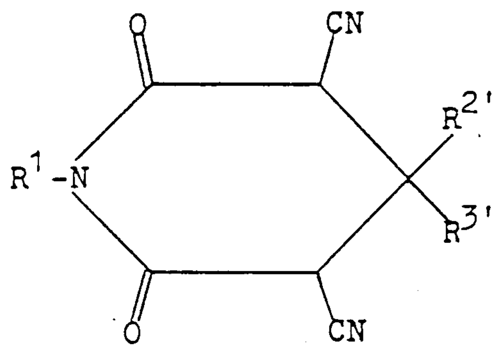

(XI)

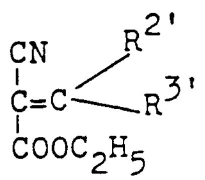

(XII)

$R^1$-NH-CO-$CH_2$-CN (XIII)

**Patentansprüche**

**1.** 3-Sulfonyl-3,7-diazabicyclo[3,3,1]nonan-Verbindungen der allgemeinen Formel I

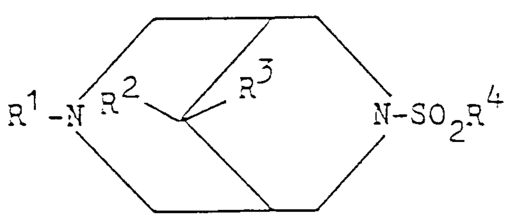

(I)

worin

$R^1$ eine Alkylgruppe mit 1-6 Kohlenstoffatomen, eine Cycloalkylalkylgruppe mit 4-7 Kohlenstoffatomen oder Benzyl bedeutet,

| | |
|---|---|
| $R^2$ | Wasserstoff oder Alkyl mit 1-4 Kohlenstoffatomen bedeutet und |
| $R^3$ | Wasserstoff oder Alkyl mit 1-4 Kohlenstoffatomen bedeutet oder |
| $R^2$ und $R^3$ | gemeinsam eine Alkylenkette mit 3-6 Kohlenstoffatomen bilden, und |
| $R^4$ | für Alkyl mit 1-4 Kohlenstoffatomen, für Thienyl, welches unsubstituiert oder durch Halogen substituiert ist, oder für eine $-(CH_2)_n-R^5$-Gruppe steht, worin n = 0-3 ist und |
| $R^5$ | eine Phenylgruppe bedeutet, welche unsubstituiert ist oder substituiert ist durch Alkyl mit 1-4 Kohnestoffatomen, Alkoxy mit 1-4 Kohlenstoffatomen, Halogen, Trifluormethyl oder Nitro, |

sowie deren Säureadditionssalze.

**2.** 3-Sulfonyl-3,7-diazabicyclo[3,3,1]nonan-Verbindungen gemäß Anspruch 1, worin $R^4$ für Thienyl, welches unsubstituiert oder durch Halogen substituiert ist, oder für eine $-(CH_2)_n-R^5$-Gruppe steht, worin n und $R^5$ die in Anspruch 1 angegebene Bedeutung besitzen.

**3.** 3-Sulfonyl-3,7-diazabicyclo[3,3,1]nonan-Verbindungen gemäß Anspruch 2, worin, $R^4$ für eine Gruppe $R^5$ steht, worin $R^5$ die in Anspruch 2 angegebene Bedeutung besitzt.

**4.** 3-Sulfonyl-3,7-diazabicyclo[3,3,1]nonan-Verbindungen gemäß den Ansprüchen 1 bis 3, worin $R^2$ und $R^3$ je Methyl bedeuten.

**5.** Arzneimittel, enthaltend eine pharmakologisch wirksame Menge einer 3-Sulfonyl-3,7-diazabicyclo[3,3,1]-nonan-Verbindung gemäß Anspruch 1 und übliche pharmazeutische Hilfs- und/oder Trägerstoffe.

**6.** Verfahren zur Herstellung von 3-Sulfonyl-3,7-diazabicyclo[3,3,1]nonan-Verbindungen der allgemeinen Formel I gemäß Anspruch 1

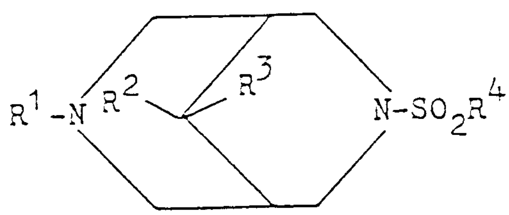

(I)

worin $R^1$, $R^2$, $R^3$ und $R^4$ die in Anspruch 1 angegebene Bedeutung besitzen, sowie deren Säureadditionssalzen,
dadurch gekennzeichnet, daß man Verbindungen der allgemeinen Formel II

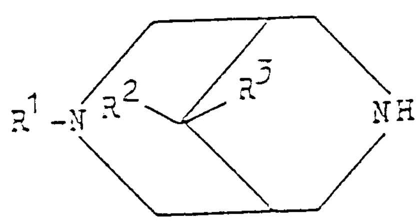

(II)

worin $R^1$, $R^2$ und $R^3$ obige Bedeutung besitzen, mit Sulfonsäurederivaten der allgemeinen Formel III

$R^4-SO_2-X$ (III)

worin $R^4$ obige Bedeutung besitzt und X eine reaktive Gruppe bedeutet, umsetzt und gewünschtenfalls freie Verbindungen der Formel I in ihre Säureadditionssalze überführt oder die Säureadditionssalze in die freien Verbindungen der Formel I überführt.

## Claims

**1.** 3-sulphonyl-3,7-diazabicyclo[3,3,1]nonane compounds of the general Formula I,

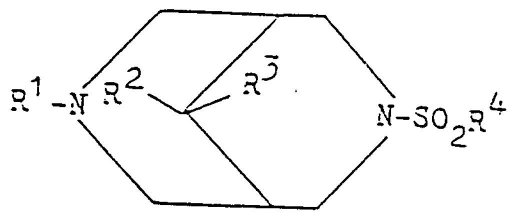

(I)

wherein

| | |
|---|---|
| $R^1$ | is an alkyl group with 1-6 carbon atoms, a cycloalkylalkyl group with 4-7 carbon atoms or benzyl, |
| $R^2$ | is hydrogen or alkyl with 1-4 carbon atoms and |
| $R^3$ | is hydrogen or alkyl with 1-4 carbon atoms or |
| $R^2$ and $R^3$ | together form an alkylene chain with 3-6 carbon atoms, and |
| $R^4$ | stands for alkyl with 1-4 carbon atoms, for thienyl which is unsubstituted or is substituted by halogen, or for a $-(CH_2)_n-R^5$ group, wherein n = 0-3 and |
| $R^5$ | is a phenyl group which is unsubstituted or is substituted by alkyl with 1-4 carbon atoms, alkoxy with 1-4 carbon atoms, halogen, trifluoromethyl or nitro, |

and their acid addition salts.

**2.** 3-sulphonyl-3,7-diazabicyclo[3,3,1]nonane compounds according to Claim 1, wherein $R^4$ stands for thienyl which is unsubstituted or is substituted by halogen, or for a $-(CH_2)_n-R^5$ group, wherein n and $R^5$ have the meanings given in Claim 1.

**3.** 3-sulphonyl-3,7-diasabicyclo[3,3,1]nonane compounds according to Claim 2, wherein $R^4$ stands for a group $R^5$, wherein $R^5$ has the meaning given in Claim 2.

**4.** 3-sulphonyl-3,7-diazabicyclo[3,3,1]nonane compounds according to Claims 1 to 3, wherein $R^2$ and $R^3$ are each methyl.

**5.** Medicaments, containing a pharmacologically effective quantity of a 3-sulphonyl-3,7-diazabicyclo[3,3,1]-nonane compound according to Claim 1 and conventional pharmaceutical auxiliaries and/or carrier substances.

**6.** A method for producing 3-sulphonyl-3,7-diazabicyclo[3,3,1]nonane compounds of the general Formula I in accordance with Claim 1,

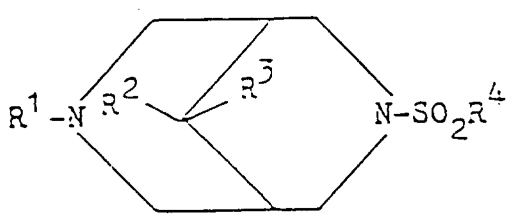

(I)

wherein $R^1$, $R^2$, $R^3$ and $R^4$ have the meanings given in Claim 1,
and their acid addition salts,
characterised in that compounds of the general Formula II,

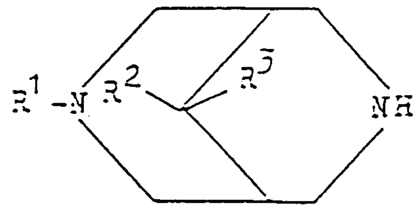

(II)

wherein $R^1$, $R^2$ and $R^3$ have the above meanings, are reacted with sulphonic acid derivatives of the general Formula III,

$R^4$-$SO_2$-X (III)

wherein $R^4$ has the above meaning and X represents a reactive group, and if desired free compounds of Formula I are converted into their acid addition salts or the acid addition salts are converted into the free compounds of Formula I.

## Revendications

**1.** Composés de type 3-sulfonyl-3,7-diazabicyclo[3.3.1]nonane de formule générale I

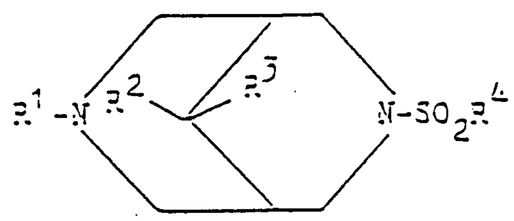

(I)

dans laquelle

| | |
|---|---|
| $R^1$ | représente un groupe alkyle ayant 1-6 atomes de carbone, un groupe cycloalkylalkyle ayant 4-7 atomes de carbone ou le groupe benzyle, |
| $R^2$ | représente un atome d'hydrogène ou un groupe alkyle ayant 1-4 atomes de carbone et |
| $R^3$ | représente un atome d'hydrogène ou un groupe alkyle ayant 1-4 atomes de carbone ou |
| $R^2$ et $R^3$ | forment ensemble une chaîne alkylène ayant 3-6 atomes de carbone, et |
| $R^4$ | représente un groupe alkyle ayant 1-4 atomes de carbone, un groupe thiényle qui n'est pas substitué ou est substitué par un halogène, ou représente un groupe $-(CH_2)_n-R^5$ dans lequel n = 0-3 et |
| $R^5$ | représente un groupe phényle qui n'est pas substitué ou est susbtitué par des substituants halogène, alkyle ayant 1-4 atomes de carbone, alcoxy ayant 1-4 atomes de carbone, trifluorométhyle ou nitro, |

ou sels d'addition avec des acides de ceux-ci.

**2.** Composés de type 3-sulfonyl-3,7-diazabicyclo[3.3.1]nonane selon la revendication 1, dans lesquels $R^4$ représente un groupe thiényle qui n'est pas substitué ou est substitué par un halogène, ou représente un groupe $-(CH_2)_n-R^5$ dans lequel n et $R^5$ ont les significations données dans la revenddication 1.

**3.** Composés de type 3-sulfonyl-3,7-diazabicyclo[3.3.1]nonane selon la revendication 2, dans lesquels $R^4$ représente un groupe $R^5$, $R^5$ ayant la signification donnée dans la revendication 2.

**4.** Composés de type 3-sulfonyl-3,7-diazabicyclo[3.3.1]nonane selon les revendications 1 à 3, dans lesquels $R^2$ et $R^3$ représentent chacun le groupe méthyle.

**5.** Médicaments contenant une quantité pharmacologiquement efficace d'un composé de type 3-sulfonyl-3,7-diazabicyclo[3.3.1]nonane selon la revendication 1, et des adjuvants et/ou véhicules pharmaceutiques usuels.

**6.** Procédé de préparation de composés de type 3-sulfonyl-3,7-diazabicyclo[3.3.1]nonane de formule générale I selon la revendication 1,

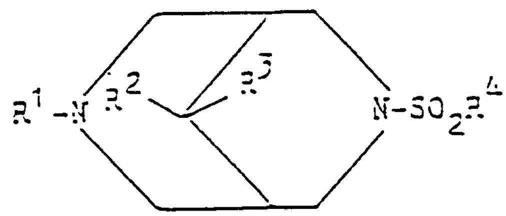

(I)

dans laquelle $R^1$, $R^2$, $R^3$ et $R^4$ ont les significations données dans la revendication 1,
ainsi que de leurs sels d'addition avec des acides, caractérisé en ce que l'on fait réagir des composés de formule générale II

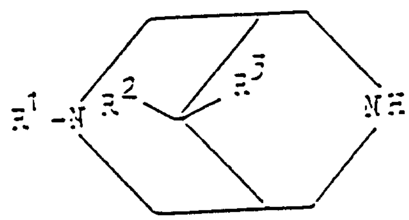

(II)

dans laquelle $R^1$, $R^2$ et $R^3$ ont les significations données plus haut, avec des dérivés d'acide sulfonique de formule générale III

$R^4$-$SO_2$-X (III)

dans laquelle $R^4$ a la signification donnée plus haut et X représente un groupe réactif, et, si on le désire, on convertit des composés de formule I libres en leurs sels d'addition avec des acides, ou on convertit les sels d'addition avec des acides en composés de formule I libres.